# EUROPEAN PATENT SPECIFICATION

(11) **EP 3 412 263 B1**
(45) Date of publication and mention of the grant of the patent: **07.06.2023**
(21) Application number: 18169402.7
(22) Date of filing: 26.04.2018
(51) Int. Cl.: A61F 13/08, B29D 28/00, B29C 48/13, D04H 3/073, D04H 3/04, D04H 3/16

(54) **SOCK FOR PATIENT USE AND METHOD OF MANUFACTURING A SOCK**
SOCKE ZUR PATIENTENVERWENDUNG UND VERFAHREN ZUR HERSTELLUNG EINER SOCKE
CHAUSSETTE POUR UTILISATION SUR UN PATIENT ET PROCÉDÉ DE FABRICATION D'UNE CHAUSSETTE

(30) Priority: 28.04.2017 FI 20175379
(43) Date of publication of application: 12.12.2018
(73) Proprietor: CareCare Oy, 80110 Joensuu (FI)
(72) Inventor: Lampio, Aino, 80400 Ylämylly (FI); Koivurova, Heikki, 80400 Ylämylly (FI)
(74) Representative: Kolster Oy Ab

(56) References cited:
- DE-B- 1 215 912
- FR-A1- 2 204 730
- FR-A1- 2 521 065
- US-A- 2 919 467
- US-A- 3 423 377

## Description

### Background

The invention relates to socks and in particular to friction-forming socks. FR 2 204 730 A1 discloses an extrusion die for making non-woven fabric which is adaptable for socks, for example.

### Brief description of the invention

The object of the invention is thus to develop a new sock for patient use and a method of manufacturing such a sock. The object of the invention is achieved with a sock and method which are characterized by what is stated in the independent claims. Preferred embodiments of the invention are disclosed in the dependent claims.

The invention is based on the sock having a tubular mesh structure, formed of single-element monofilaments and flexible in its transverse direction, at least.

Benefits of the solution include the ability of the sock to breath, and a good fit to feet of different sizes, and simple manufacturability.

### Brief description of the figures

The invention will now be described in more detail in connection with preferred embodiments and with reference to the accompanying drawings, in which:
Figure 1 is a schematic view of a sock for patient use;
Figure 2 is a schematic view of a mesh structure;
Figures 3a and 3b are schematic views of socks for patient use;
Figures 4a to 4e show cross sections of monofilaments;
Figure 5 is a schematic representation of a method; and
Figure 6 is a schematic view of a nozzle arrangement.

### Detailed description of the invention

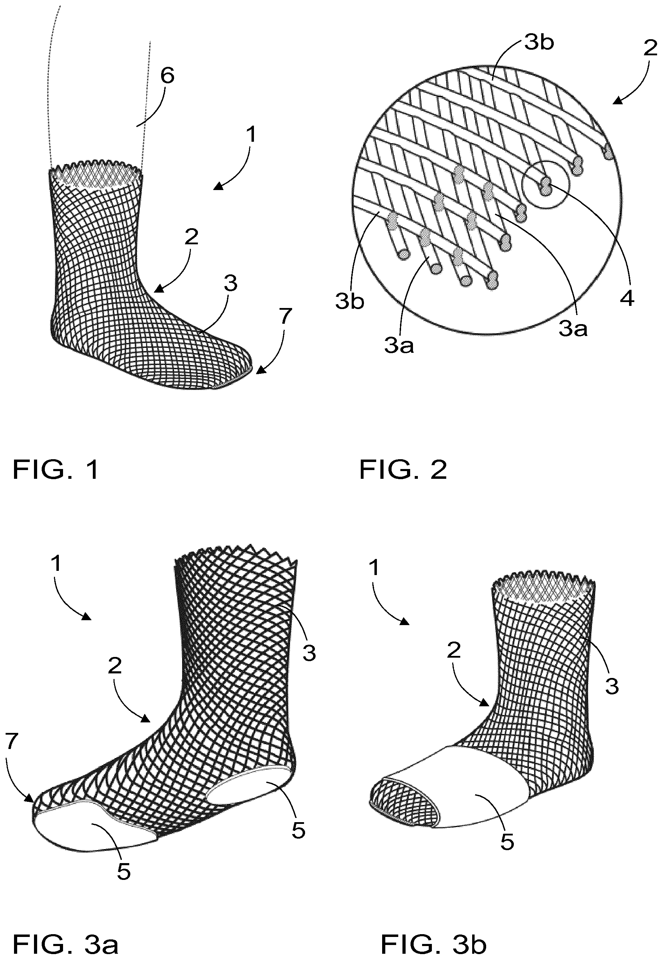

Figure 1 is a schematic view of a sock 1 for patient use. Patient use may comprise the use of the sock by a patient at home care, hospital care, or care at another similar facility. Usage situations may vary, comprising bed rest, sitting, and/or moving of the patient either alone or aided. Obviously, the sock now disclosed is suitable for other purposes as well and is not restricted to the above usage situations.

The sock 1 for patient used comprises a tubular mesh structure 2, such as a mesh structure 2 of the type shown schematically in Figure 2. The mesh structure 2 is breathable due to its very structure and consequently it is not sweaty.

The mesh structure 2 is flexible at least in its transverse direction, that is, in the direction transverse in relation to the longitudinal direction of the tube. In such a case, the mesh structure is adaptable in a fitting way, forming to the shapes of a foot but not compressing them, for feet of different sizes and is suitable, for example, to be simultaneously used with patient clothes or means of treatment of various kinds. In an embodiment, the mesh structure 2 may be formed to be flexible in its longitudinal direction, too.

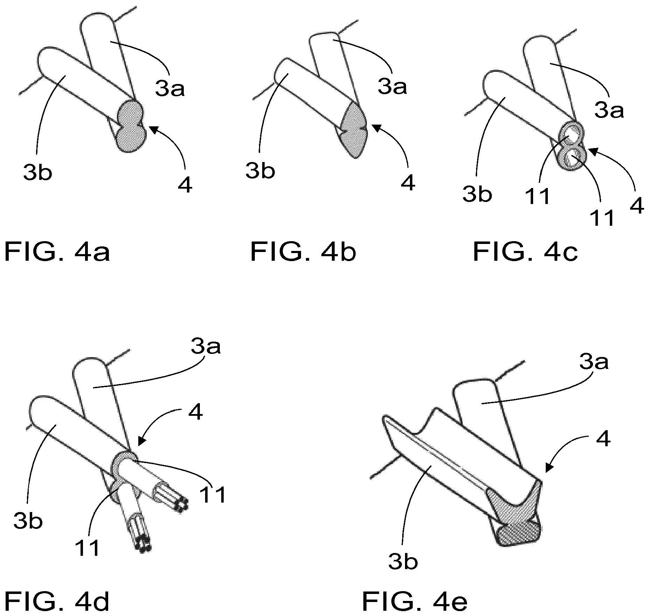

The mesh structure 2 comprises monofilaments 3. In other words, the mesh structure 2 is formed of single-fibre, yarn-like parts. The monofilaments 3 may be monolithic, that is, single-element ones. In other words, the monofilaments 3 in this case do not comprise a plurality of intertwined fibres of possibly different materials, or nested or adjacent layers of materials, but consists of one fibre that is of the same uniform material through the entire cross section of the monofilament. The monofilament 3 shown in Figure 2 has a round cross section, but in different embodiments the cross section of the monofilament 3 may also be oblong, square, polygon, or of another appropriate form. Figures 4a-4e show cross sections of monofilaments 3. Figure 4a shows a monofilament 3a of the first layer and 3b of the second layer, substantially round as to their cross section. Figure 4b shows a monofilament 3a of the first layer and 3b of the second layer, substantially triangular as to their cross section. In an embodiment, the cross section of the monofilament 3 may be chosen such that is directs a massaging effect on a foot.

In an embodiment, the monofilament 3 may be adapted tubular by arranging a channel 11 inside it, as in the embodiments of Figures 4c and 4d. In other words, the monofilament 3 may be hollow on the inside. Even though the cross section of the filament in Figure 4d is substantially round, it is obvious for a person skilled in the art that the cross section of the monofilament may also vary in embodiments in which a channel 11 is arranged within the monofilament.

In an embodiment, mesh structure 2 may comprise fibre mass. The fibre mass may comprise a material in which fibres have been mixed with or into the monolithic material. Fibre mass in this case does not refer to a fibre filament in which fibres have been twisted or twined to each other or, for example, adapted into substantially parallel fibre bundles, but material in which cut fibres are mixed into the rest of the material mixture. Embodiments comprising fibre mass are suited to usages where it is necessary to strengthen the mesh structure against, for example, incisions by the means of treatment or other external pieces, and in which the mesh structure as such allows an adequate degree of flexibility.

In an embodiment, in the channel 11 arranged inside the monofilament 3, other material may have been adapted than the material of the monofilament. In an embodiment, in the channel arranged inside the monofilament 3, fibre mass, for example, may be adapted. In a second embodiment, the fibre mass may have been mixed into the monolithic material. In yet another embodiment, in the channel 11 arranged inside the monofilament 3, coated fibres, or fibres adapted inside a second monolithic material may have been adapted, as in Figure 4d. Even in such a case, it is advantageous that it is specifically the monofilaments 3a of the first layer and the monofilaments 3b of the second layer of the mesh structure 2 that are welded together on the surfaces facing each other at intersecting points 4. In other words, fibres or fibre mass possibly adapted inside the monofilament 3 form a filler and do not extend to the welding.

The monofilaments 3 are adapted in at least two layers so that the monofilaments 3 of each layer are adapted mutually substantially parallel and crosswise 3 in relation to the monofilament of the subsequent layer. In other words, the mesh structure 2 comprises a plurality of mutually substantially parallel first-layer monofilaments 3a adapted in the first layer, and a plurality of mutually substantially parallel second-layer monofilaments 3b adapted in the second layer. Each monofilament 3a of the first layer is adapted in each particular case to intersect at least one second-layer monofilament 3b. The intersecting points of the monofilaments 3, 3a, 3b of two different layers form intersecting points 4. Each monofilament 3, 3a, 3b intersects the monofilament of the subsequent layer in at least one intersecting point 4. Outside the intersecting points 4 both the monofilaments of the same layer and different layers may advantageously move in relation to each other, advantageously in a substantially free manner.

The monofilament 3, 3a, 3b may advantageously be welded to the intersecting monofilament in the subsequent layer. In other words, the monofilament 3a of the first layer may, in the areas on the side of the second layer, be solidly welded together to an area of the monofilament 3b on the side of the first layer at the intersecting point 4. In other words, the surfaces of at least monofilaments 3a, 3b are in such a case fused together in the intersecting area of the monofilaments 3a, 3b, facing each other, The welding may advantageously be achieved by forming the mesh structure 2 by an extrusion method by means of two nozzles adapted one inside the other and rotating in opposite directions, whereby each nozzle forms the monofilaments of one layer in each particular case. In such a case, the mesh structure 2 may be established at one work stage without specific moulds or similar special tools, except for the nozzles, which speeds up and simplifies the manufacturing process. At the same time, coating and other work stages related to coatings and similar manufacturing methods may be avoided.

In an embodiment, the sock 1 may be formed entirely as a single element one. In this case, the sock 1 does not comprise any separate material layers or material fibres connected to each other in one way or another, but is formed in its entirety out of the same material. In an embodiment, the sock 1 may be entirely formed as a mesh structure 2 formed by monofilaments. In such a case, the sock 1 does not comprise any separate parts, or parts attached to the sock, differing from the mesh structure 2 as to their structure. In an embodiment, the sock 1 may be entirely formed as a mesh structure 2 formed by single-element monofilaments.

The monofilament 3 is formed of a material with a high friction coefficient. In connection with this description, a high friction coefficient means that the monofilament 3 is formed of a material that has a higher friction coefficient in relation to the common floor surface materials, in other words, a higher static coefficient of friction and/or kinetic coefficient than the common sock materials such as cotton, wool, polyamide, acryl, or their combinations, typically formed as knitted fabrics of various kinds. The sock 1 may in such a case act as a so-called friction sock, which may prevent a patient from slipping when he is getting onto his feet, is walking alone or aided, or when a patient is lifted. The sock 1 acts as an anti-slip device without coating or other post-processing such as dip treatment. The socks 1 described in this description are especially usable as friction socks also when used simultaneously with patient clothes and means of treatment of various kinds, because due to their formability they are easy to fit in feet of different sizes and, for example, over conventional patient socks or even over treatment means.

In an embodiment, the mesh structure 2 may comprise thermoplastic elastomer, such as polypropylene-based thermoplastic elastomer. In an embodiment, the mesh structure 2 may comprise synthetic rubber material such as styrene butadiene rubber (SBR).

In an embodiment, the mesh structure 2 may comprise monofilaments 3 comprising different two materials so that the first-layer monofilaments 3a may comprise a different material than the second-layer monofilaments 3b. In an embodiment, the monofilaments 3 of the layer against the foot in the usage position, such as first-layer monofilaments 3a, may comprise a material whose friction coefficient is lower than that of the material that the second-layer monofilaments 3b comprise. In this manner, it is possible to form a sock 1 that is easy to put on the foot and which at the same time creates an efficient anti-slip effect between the foot and base. Such as mesh structure 2 may advantageously be formed by the extrusion method by means of two nozzles adapted one inside the other and rotating in opposite directions, whereby each nozzle forms the monofilaments of one layer at any one time and whereby the material fed to the first nozzle 8a differs from the material fed to the second nozzle 8b. In an embodiment the first-layer monofilaments 3a may comprise, for example, polypropylene and the second-layer monofilaments 3b may comprise, for example, polypropylene filled with EPDM rubber (Ethylene Propylene Diene Monomer rubber). In such a case, the inner layer may be formed of slippery and thus easy to put on and well moisture-transferring polypropylene, and the outer surface of a material that has a higher friction coefficient, and which may, however, due to the same base material may be easily adapted to weld to each other.

In an embodiment, structures welding to each other, such as the first-layer monofilaments 3a and the second-layer monofilaments 3b or the monofilaments 3, 3a, 3b of the mesh structure 2 and a slide block 5, comprise one or more monolithic material that have the same base substance. In such embodiments, it is easy to arrange the welding of the structures to each other. As disclosed in the other embodiments, the welding structures may in such a case comprise the same material or alternatively materials that differ from each other, but which, however, advantageously have the same matrix whereby they weld together but may nevertheless achieve different properties on different surfaces.

In an embodiment, the mesh structure 2 may comprise two monofilaments 3a, 3b having different cross sections so that the first-layer monofilaments 3a may comprise a cross section of a different shape than the second-layer monofilaments 3b. In an embodiment, the monofilaments 3 of the layer against the foot in the usage position, such as first-layer monofilaments 3a, may comprise a cross section whose friction coefficient is lower than the cross section that the second-layer monofilaments 3b comprise. An example of such an embodiment is shown in Figure 4e. In this case, the shape of the surface of the first-layer monofilaments 3a towards the foot in the usage position may be formed smooth and/or narrow, such as rounded. Correspondingly, the shape of the surface of the second-layer monofilaments 3b towards the base in the usage position may be formed to add to the friction and/or friction surface, such as grooved and/or wider than the surface of the first-layer monofilaments 3a, directed towards the foot. In this manner, it is possible to form a sock 1 that is easy to put on the foot and which at the same time creates an efficient anti-slip effect between the foot and base. Such as mesh structure 2 may advantageously be formed by the extrusion method by means of two nozzles adapted one inside the other and rotating in opposite directions, whereby each nozzle forms the monofilaments of one layer at any one time and whereby the cross section of openings 10 formed in the first nozzle 8a differs from the cross section of openings 10 formed in the second nozzle 8b.

In an embodiment, the sock 1 may comprise silver ions to establish an anti-bacteria effect. So, in this case the sock 1 may be formed anti-bacterial. This is beneficial in connection with wounds and ulcers of various kinds and in hospital and similar facility environments, in particular. By means of silver ions, the sock 1 may also be formed as one removing body odour.

The tubular mesh structure 2 is formed to be closed at one end so that the sock forms a bag-like mesh structure. Such a sock 1 may be formed, for example, by forming a tubular mesh structure 2 by the extrusion method with two nozzles adapted one inside the other and rotating in opposite directions, and by closing one end of the tubular mesh structure 2 by melting or welding, for example.

Figures 3a and 3b are schematic views of socks 1 for patient use. In an embodiment, such as in the embodiments of Figures 3a and 3b, the sock 1 may comprise at least one slide block 5. The slide block 5 is formed of a material having a friction coefficient lower than the friction coefficient of the mesh structure 2. In such a case, the sock 1 may be formed on a chosen area, that is, in the area of the slide block 5, to be better sliding than elsewhere in the area of the sock 1, such as the area of the mesh structure 2. Depending on the embodiment, the slide block 5 may comprise a film-like or mesh-like piece. In an embodiment, one slide block 5 or a plurality of slides 5 may be fixedly adapted in the mesh structure 2 by welding. In a second embodiment, one slide block 5 or a plurality of slides 5 may be adapted in the mesh structure 2 by seaming. In a third embodiment, one slide block 5 or a plurality of slides 5 may be adapted in the mesh structure 2 by laminating. In other words, at least one slide block 5 may be adapted in the mesh structure immovably and non-detachably by, for example, welding, seaming, or laminating.

In an embodiment, at least one slide block 5 may be adapted in the sock 1 so that the slide block 5 places itself under the front part of the foot when the sock 1 has been put on a leg 6. In an embodiment, the mesh structure 2 may be formed closed at one of its ends, and the slide block 5 in such a case may be adapted in the mesh structure 2 closer to the closed end 7 of the mesh structure 2 than its open end, whereby the slide block 5 places itself under the front part of the foot when the sock has been put on the leg 6. Such a sock 1 is most advantageous in uses where the sock is on the one hand required to be sliding and on the other hand to have friction. Such a situation may exists for a person who has restricted movement of one or both of his legs, such as the ability to raise the foot into the air while walking, in which case the person himself or aided may slide the foot on a base by, for example, moving the heel up. In an embodiment, at least one slide block 5 may be adapted in the sock 1 so that it extends substantially around the sock, advantageously in the area that places itself under the front part of the foot when the sock 1 has been put on the leg 6, such as in the embodiment of Figure 3b. The advantage of such a sock 1 is that it does not matter which way or in which position the sock is put on the leg.

In the embodiment of Figure 3a, the sock 1 comprises two slides 5. In a second embodiment, the sock 1 may comprise, as stated in the above, just one slide block 5, such as the slide block 5 of Figure 3a, arranged on the left, so in connection with the closed end of the sock 1, in other words under the front part of the foot.. In an embodiment, a second slide block 5 may be adaptable to be placed under the heel according to Figure 3a. In such a case, the slide block arranged under the heel may, for example, in an embodiment comprise material that has a higher friction coefficient that the slide block 5 adaptable under the front part of the foot.

In an embodiment, the sock 1 comprises a separate flexible cuff. The flexible cuff may be formed of the same material as the mesh structure 2 or of a different material. Such a flexible cuff may facilitate putting on the sock 1 and helps the sock stay on the leg. The flexible cuff may be fixedly adapted in the open end of the mesh structure by welding, for example.

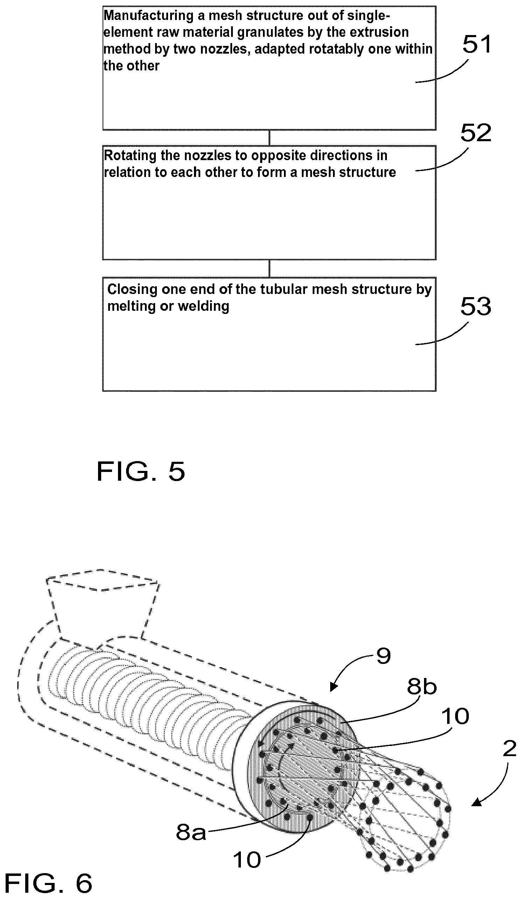

Figure 5 is a schematic representation of a method for making a sock 1 for patient use, such as any of the socks 1 disclosed in this description. Such a sock 1 may be manufacturing 51 the mesh structure 2 out of single-element raw material granulates by the extrusion method. The mesh structure 2 may be made by two nozzles 8a, 8b arranged one within the other, such as by the nozzle arrangement 9 schematically shown in Figure 5a or 5b, comprising such nozzles 8a, 8b. The nozzles 8a, 8b may be adapted rotatable in relation to each other. Advantageously the nozzles 8a, 8b may be adapted to rotate in opposite directions so that, for example, the inner nozzle 8a rotates clockwise and the outer nozzle 8b rotates anticlockwise, such as in Figure 6, which is a schematic view of a nozzle arrangement, or correspondingly so that the inner nozzle 8a rotates anticlockwise and the outer nozzle 8b rotates clockwise. In both nozzles 8a, 8b, openings 10 for the extrusion material may have been formed circumferentially at a distance from each other. In such a case, the nozzles 8a, 8b may, in other words, be rotated 52 in opposite directions in relation to each other to form the tubular mesh structure 2. In other words, extrusion material may be led through the openings 10 as the nozzles 8a, 8b are rotated in opposite directions in relation to each other to form the tubular mesh structure 2. A bag-like sock, closed at one end, is formed 53 by closing one end of the tubular mesh structure by melting or welding, for example.

In an embodiment, the mesh structure 2 may be formed by any of the methods disclosed in this description, whereby the mesh structure may be used for other purposes than to make a sock intended for patient use.

In different embodiment, the method may further comprise features and process steps described in connection with the embodiments 2 and socks 1, such as the use of different materials and openings 10, adapting at least one slide block 5 in the mesh structure 2 and/or sock 1, and so forth. Correspondingly, in the manufacture of the discloses mesh structures 2 and socks 1, a method of any of the described embodiments may be used.

Ordinary patient socks are typically knit tubes, sewn closed at an end, which have the tendency to slip and stay badly in place. The sock according to the solution now put forth may, for example, be adapted over such a patient sock, in which case it keeps the patient sock firmly in place without compressing, and on the other hand acts as an anti-slip device as the patient is moving alone or aided. Because the sock now put forth does not compress the foot, it is suitable for use on feet that hurt and/or are swollen. In addition, If need be, the sock may be easily modified according to patient-specific needs by, for example, cutting the mesh structure open where needed, such as at a wound or a swelling, or to adapt means of treatment to pass through the sock.

A sock according so some of the embodiments disclosed in this description may also be washed, allowing it to be used for a number of times, in other words it is not for a single use, only. This reduces the amount of garbage produced.

Even though the mesh structure 2 and sock 1 now described are particularly well suited for patient use, it is obvious for a person skilled in the art that the mesh structure 2 and sock 1 may also be used for other purposes where a tubular mesh structure flexible at least in the transverse direction and cost-effective to manufacture is needed. To take an example, the embodiments provided with the friction characteristics may be used, in addition to patient use, by elderly people and, for example, in bathroom, swimming hall, or spa conditions to prevent slipping.

A person skilled in the art will find it obvious that, as technology advances, the basic idea of the invention may be implemented in many different ways. The invention and its embodiments are thus not restricted to the above-described examples but may vary within the scope of the claims.

## Claims

1. Sock for patient use, the sock comprising
a tubular mesh structure flexible at least in its transverse direction such that a foot of the patient is not compressed, the tubular mesh structure comprising
single-element monofilaments, adapted in at least two layers so that the monofilaments of each layer are adapted mutually substantially parallel and crosswise in relation to the monofilament of the subsequent layer, and each monofilament is at each intersecting point where the monofilament intersects the monofilament of the subsequent layer welded to the intersecting monofilament of the subsequent layer,
the monofilament being formed of a material with a high friction coefficient which high friction coefficient means that the monofilament is formed of a material that has a higher friction coefficient in relation to the common floor surface materials, in other words, a higher static coefficient of friction and/or kinetic coefficient than the common sock materials such as cotton, wool, polyamide, acryl, or their combinations, typically formed as knitted fabrics of various kinds, and
the tubular mesh structure being formed to be closed at one end by closing the tubular mesh structure at one end by melting or welding so that the sock forms a bag-like mesh structure,
wherein the material with a high friction coefficient comprises synthetic rubber material and/or thermoplastic elastomer, and
wherein the sock is entirely formed of a mesh structure formed by said single-element monofilaments or
the sock further comprising a slide block formed of a material possessing a lower friction coefficient than the friction coefficient of the mesh structure, and being fixedly adapted to the mesh structure by welding.

2. A sock as claimed in claim 1, wherein the synthetic rubber material comprises styrene butadiene rubber.

3. A sock as claimed in claim 1 or 2, comprising silver ions to establish an anti-bacteria effect.

4. A sock as claimed in any one of claims 1 to 3, which further comprises a separate cuff which is fixedly adapted to the open end of the mesh structure by welding.

5. A method for manufacturing a sock for patient use, in which method a sock as claimed in any one of claims 1 to 4 is manufactured by manufacturing a tubular mesh structure out of single-element raw material granulates by the extrusion method by two nozzles adapted rotatably one within the other, to both of which openings for the extrusion material have been adapted circumferentially at a distance from each other so that the nozzles are rotated in opposite directions in relation to each other to form the tubular mesh structure, and closing the other end of the tubular mesh structure by melting or welding.

## Patentansprüche

1. Socke zur Patientenverwendung, wobei die Socke Folgendes umfasst:
eine rohrförmige Maschenstruktur, die zumindest in ihrer Querrichtung derart biegsam ist, dass ein Fuß des Patienten nicht zusammengedrückt wird, wobei die rohrförmige Maschenstruktur Folgendes umfasst:
Einzelelement-Monofilamente, die derart in mindestens zwei Schichten angepasst sind, dass die Monofilamente von jeder Schicht gegenseitig im Wesentlichen parallel und kreuzweise in Bezug auf das Monofilament der anschließenden Schicht angepasst sind, und jedes Monofilament sich an jedem Schnittpunkt befindet, wo das Monofilament das Monofilament der anschließenden Schicht schneidet, das an das schneidende Monofilament der anschließenden Schicht geschweißt ist,
wobei das Monofilament aus einem Material mit einem hohen Reibungskoeffizienten gebildet ist, wobei der hohe Reibungskoeffizient bedeutet, dass das Monofilament aus einem Material gebildet ist, das im Verhältnis zu den üblichen Bodenoberflächenmaterialien einen höheren Reibungskoeffizienten, mit anderen Worten einen höheren statischen Reibungskoeffizienten und/oder kinetischen Koeffizienten, als die üblichen Sockenmaterialien, wie beispielsweise Baumwolle, Wolle, Polyamid, Acryl oder ihre Kombinationen, die typischerweise als gestrickte Gewebe verschiedener Arten gebildet sind, aufweist, und
die rohrförmige Maschenstruktur gebildet ist, um an einem Ende durch Schließen der rohrförmigen Maschenstruktur an einem Ende durch Schmelzen oder Schweißen geschlossen zu werden, derart dass die Socke eine taschenartige Maschenstruktur bildet,
wobei das Material mit einem hohen Reibungskoeffizienten Synthesekautschukmaterial und/oder thermoplastisches Elastomer umfasst, und
wobei die Socke vollständig aus einer Maschenstruktur gebildet ist, die aus den Einzelelement-Monofilamenten gebildet ist, oder
die Socke ferner einen Gleitblock umfasst, der aus einem Material gebildet ist, das einen niedrigeren Reibungskoeffizienten als der Reibungskoeffizient der Maschenstruktur besitzt und durch Schweißen fest an die Maschenstruktur angepasst ist.

2. Socke nach Anspruch 1, wobei das Synthesekautschukmaterial Styrol-Butadien-Kautschuk umfasst.

3. Socke nach Anspruch 1 oder 2, die Silberionen umfasst, um eine antibakterielle Wirkung herzustellen.

4. Socke nach einem der Ansprüche 1 bis 3, die ferner einen separaten Bund umfasst, der durch Schweißen fest an das offene Ende der Maschenstruktur angepasst ist.

5. Verfahren zur Herstellung einer Socke zur Patientenverwendung, wobei in dem Verfahren eine Socke nach einem der Ansprüche 1 bis 4 durch Herstellen einer rohrförmigen Maschenstruktur aus Einzelelement-Rohmaterialgranulaten durch das Extrusionsverfahren durch zwei Düsen, die ineinander drehbar angepasst sind, wobei beide ihrer Öffnungen für das Extrusionsmaterial derart in Umfangsrichtung in einem Abstand voneinander angepasst wurden, dass die Düsen in zueinander entgegengesetzten Richtungen gedreht werden, um die rohrförmige Maschenstruktur zu bilden, und Schließen des anderen Endes der rohrförmigen Maschenstruktur durch Schmelzen oder Schweißen hergestellt wird.

## Revendications

1. Chaussette destinée à être utilisée par un patient, la chaussette comprenant :
une structure en maille tubulaire flexible au moins dans sa direction transversale de façon que le pied du patient ne soit pas comprimé, la structure en maille tubulaire comprenant
des monofilaments à un seul élément, adaptés dans au moins deux couches de façon que les monofilaments de chaque couche soient adaptés mutuellement pratiquement parallèles et transversaux en relation avec le monofilament de la couche suivante, et que chaque monofilament soit à chaque point d'intersection où le monofilament croise le monofilament de la couche suivante soudé au monofilament en intersection de la couche suivante,
le monofilament étant formé d'un matériau ayant un coefficient de frottement élevé, lequel coefficient de frottement élevé signifie que le monofilament est formé d'un matériau qui a un coefficient de frottement plus élevé en relation avec les matériaux de surface de sol courants, en d'autres termes un coefficient de frottement statique et/ou un coefficient cinétique supérieur à celui des matériaux de chaussette courants tels que le coton, la laine, le polyamide, l'acrylique, ou leurs combinaisons, typiquement formés en étoffes tricotées de divers types, et
la structure en maille tubulaire étant formée de façon à être fermée à une extrémité par fermeture de la structure en maille tubulaire à une extrémité par fusion ou soudage de façon que la chaussette forme une structure en maille en forme de sac,
dans laquelle le matériau ayant un coefficient de frottement élevé comprend un matériau en caoutchouc synthétique et/ou un élastomère thermoplastique, et
laquelle chaussette est entièrement formée d'une structure en maille formée par lesdits monofilaments à un seul élément, ou
la chaussette comprenant en outre un patin de glissement formé d'un matériau possédant un coefficient de frottement inférieur au coefficient de frottement de la structure en maille, et adapté de façon fixe à la structure en maille par soudage.

2. Chaussette selon la revendication 1, dans laquelle le matériau en caoutchouc synthétique comprend un caoutchouc de styrène-butadiène.

3. Chaussette selon la revendication 1 ou 2, comprenant des ions argent pour établir un effet antibactérien.

4. Chaussette selon l'une quelconque des revendications 1 à 3, qui comprend en outre un revers séparé qui est adapté de façon fixe à l'extrémité ouverte de la structure en maille par soudage.

5. Procédé pour fabriquer une chaussette destinée à être utilisée par un patient, procédé dans lequel une chaussette selon l'une quelconque des revendications 1 à 4 est fabriquée par fabrication d'une structure en maille tubulaire à partir de granulats de matières premières à un seul élément par un procédé d'extrusion au moyen de deux buses adaptées de façon à tourner l'une dans l'autre, des ouvertures pour le matériau d'extrusion ayant été adaptées à ces deux buses de manière circonférentielle à une certaine distance mutuelle de façon que les buses tournent dans des directions opposées l'une par rapport à l'autre pour former une structure en maille tubulaire, et par fermeture de l'autre extrémité de la structure en maille tubulaire par fusion ou soudage.
